(19) Europäisches Patentamt
European Patent Office
Office européen des brevets

(11) EP 1 452 175 A1

(12) **EUROPEAN PATENT APPLICATION**

(43) Date of publication:
**01.09.2004 Bulletin 2004/36**

(51) Int Cl.[7]: **A61K 31/28**, A61P 39/06

(21) Application number: **03290491.4**

(22) Date of filing: **28.02.2003**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(71) Applicant: **Medesis Pharma S.A.**
**75002 Paris (FR)**

(72) Inventors:
- **Maurel, Jean-Claude**
**34160 Castries (FR)**
- **Cudennec, Claude-Alain**
**76230 Bois-Guillaume (FR)**

(74) Representative: **Tezier Herman, Béatrice et al**
**Cabinet Becker & Associés**
**35, rue des Mathurins**
**75008 Paris (FR)**

(54) **Manganese based organometallic complexes, pharmaceutical compositions and dietetic products**

(57) The invention relates to compounds and their uses, particularly in the pharmaceutical and dietetic industries. The invention discloses complexes having Mn-SOD like activities, as well as methods for treating various diseases associated with oxidative stress, including cancer and inflammatory conditions, by administering said compounds. It further deals with pharmaceutical compositions or dietetic products comprising said compounds, more particularly useful to treat various diseases or disorders, in particular useful in the prevention or treatment of a disease involving an oxidative stress.

EP 1 452 175 A1

## Description

The invention relates to compounds and their uses, particularly in the pharmaceutical and dietetic industries. The invention discloses complexes having Mn-SOD like activities, as well as methods for treating various diseases associated with oxidative stress, including cancer and inflammatory conditions, by administering said compounds. It further deals with pharmaceutical compositions or dietetic products comprising said compounds, more particularly useful to treat various diseases or disorders, in particular useful in the prevention or treatment of a disease involving an oxidative stress.

## BACKGROUND OF THE INVENTION

One vital feature of manganese, which is not widely appreciated, is its role as an essential element in maintaining human health. Recommended daily dietary intake levels have been established by US regulatory authorities in an effort to ensure the maintenance of good health. The exact role of manganese is not fully understood, but complex cellular reactions involving metallo-enzymes have been identified as the potential mechanism to explain its role. Humans have well-developed homeostatic control mechanisms whereby manganese levels are regulated to keep them in the desired range. Medical research into conditions arising from an excess or deficit of body manganese is being carried out in a number of laboratories.

There is general agreement within the health professionals and dieticians that manganese is essential to ensure the health and well being of humans and animals. The human body contains from 12 to 20 milligrams of manganese. Estimations of the human requirements for manganese vary considerably, but are based on studies of the balance between intake and excretion necessary to maintain this level. Data from several studies suggest that manganese intake of from 0.035 to 0.070 milligrams per kilo of body weight per day provides the needed balance. However, a 1988 study conducted by researchers at the University of Texas at Austin found that a minimum of 3.5 milligrams per day seems necessary.

Human consumption depends on the amount of certain foods consumed. The typical English winter diet (with substantial tea intake) provides up to 8.8 mg of manganese per day, while studies of women in Japan, Canada, New Zealand and the USA suggest average daily intakes from 2.5 to 4 mg per day.

Manganese deficiency has been demonstrated in animals and has been noted in humans in association with vitamin K deficiency. Its main manifestations in all species studied are impaired growth, skeletal abnormalities, disturbed or depressed reproductive functions, lack of muscular coordination among newborns and defects in lipid and carbohydrate metabolisms.

The following fresh food groups (in descending order) are most important in manganese content: nuts, whole cereals, dried fruits, roots, tubers and stalks, fruits, non-leafy vegetables, meat, poultry products, fish and seafoods. Leafy vegetables also rank high on the list when expressed in dry-weight terms. Tea has a very high manganese content, ten times that of cereals. The wide range of manganese contained in cereal grains and products depends on the species and on the milling process used. In a US study, wholewheat containing 31 ppm total manganese yielded 160 ppm in the germ, 119 ppm in the bran and only 5 ppm in the white flour. Half a cup of oatmeal, 30 grams of shredded wheat or raisin bran cereal, a quarter of a cup of pecans or a third of a cup of peanuts, and half a cup of cooked spinach, contain each over a milligram of manganese. Sweet potatoes, red lima or navy beans and pineapple juice are other sources. There is very little or no manganese present in dairy products and highly refined sugar-containing foods.

Primary reactive oxygen species (ROS) such as superoxide radical, hydrogen peroxide, hydroxyl radicals, and ortho-quinone derivatives of catecholamines exert their cellular effects by modifying DNA, lipids, and proteins to form secondary electrophiles. The secondary electrophiles are implicated in cellular dysfunction either because they are no longer able to participate in normal cellular activity or because they serve as electron acceptors in oxidative chain reactions that result in the modification of other essential cellular components. Damage caused by the primary and secondary ROS contributes to the pathogenesis of important human diseases. In particular, one consequence of oxidative metabolism is the generation of superoxide radicals ($O_2^-$) which mediate extensive damage to the cellular components of living organisms. The molecular dismutation of $O_2^-$ to hydrogen peroxide ($H_2O_2$) and oxygen ($O_2$) is catalysed by a ubiquitous class of metalloenzymes termed superoxide dismutases (SODs). The prevalence of SODs in all living organisms which tolerate exposure to molecular $O_2$ has led to the compelling suggestion that these enzymes form the first line of the cell's defence against oxygen damage. Indeed, mice defective for SOD do not survive and reduction of functional capabilities of this enzyme generates an high increase of oxidative stress in connection with strong mitochondrial disabilities of cells.

On the basis of their metal ion content, three classes of SOD are recognised: Cu/Zn-, Fe-, and Mn-containing enzymes. While all three forms catalyse the same reaction, the Fe-containing SODs (FeSOD) are largely confined to prokaryotes and the Cu/Zn enzymes (Cu/ZnSOD) predominantly to eukaryotes. Mn-containing SODs (MnSOD) are universally present. In eukaryotes MnSODs are localised to the mitochondria, while the Cu/ZnSODs reside in the

cytosol.

The superoxide radical ($O_2^-$) can be generated within living cells during both enzymic and non-enzymic oxidations. Because of the direct reactivity of $O_2^-$, and the reactivity of secondary free radicals that it can generate, $O_2^-$ presents a threat to cellular integrity. This threat is met by a family of defensive enzymes that catalyze the conversion of $O_2^-$ to $H_2O_2 + O_2$. These enzymes, superoxide dismutases (SOD), react with $O_2^-$ at a rate that approaches the theoretical diffusion limit and appear to be important for aerobic life. The $H_2O_2$ generated by SOD is disposed of either by catalytic conversion to $O_2$ and $H_2O$ by catalases, or by reduction to water at the expense of thiol, amine or phenolic substrates by peroxidases.

The superoxide radical has been shown to be an important causative factor in the damage resulting from: autoxidation; oxygen toxicity; the oxygen-dependent toxicity of numerous compounds; reperfusion injury; inflammation; and frostbite; and is implicated in the limited viability of transplanted organs and tissues.

The earliest work bearing on the functions of SOD dealt primarily with oxygen toxicity and with the oxygen-dependent toxicities of viologens, quinones and related redox-cycling compounds. These investigations established that $O_2^-$, made within cells, can kill the cells and that SOD provides a defense. It is now known that $O_2^-$ is not only an unwanted and dangerous by-product of dioxygen metabolism, but is also produced in large quantities by certain specialized cells, seemingly to serve a specific purpose. Neutrophils, and related phagocytic leucocytes, contain a membrane-associated NADPH oxidase that is activated when the cells are stimulated and that specifically reduces dioxygen to $O_2^-$. A defect in this enzyme weakens the microbicidal activity of these leucocytes, leading to chronic granulomatous disease.

The known association of neutrophils with the inflammatory process, and the production of $O_2^-$ by activated neutrophils, suggests a role for $O_2^-$ in the development, and possibly in the deleterious consequences, of inflammation. An enzymic source of $O_2^-$ decreases the viscosity of synovial fluid by depolymerizing hyaluronate and SOD exerts a protective effect. Injecting an enzymic source of $O_2^-$, such as xanthine oxidase, causes a localized inflammation that can be prevented by scavengers of oxygen radicals, such as SOD.

The anti-inflammatory effect of SOD, noted in model inflammations in laboratory animals, is explained in terms of the inhibition of the production of a neutrophil chemotaxin by the reaction of $O_2^-$ with a precursor present in normal human serum. SOD, when injected into the circulation, is rapidly removed by the kidneys, such that the circulation half life of i.v.-injected bovine SOD in the rat is only 7 minutes. This can be markedly increased by coupling the SOD to polyethylene glycol or ficoll, with a corresponding increase in anti-inflammatory effect.

The tissue damage that develops as a consequence of temporary ischemia has classically been attributed to the lack of ATP which develops during the hypoxia imposed during ischemia. Data support the view that this damage actually occurs during reperfusion and is an expression of increased oxygen radical production. SOD protects against this reperfusion injury.

SODs from various sources are currently of great interest as potential therapeutic treatments for oxidative damage. Their use in a clinical setting for the treatment of a wide variety of disorders has been proposed. Generally, the superoxide dismutases are credited with a protective function against certain inflammatory processes. They have been investigated in the cases of the reperfusion injury associated with skin grafts, organ transplants, frostbite and myocardial infarction. In particular, deficiency in Mn-SOD is supposed to have some significance in the development of rheumatoid arthritis (Pasquier, C. et al., Inflammation 8, 27-32, 1984). SOD is also assumed to have a protective effect against alcohol-induced liver damage (Del Villano B. C. et al., Science 207, 991-993, 1980).

Additional potential therapeutic effects for SOD include: (i) prevention of oncogenesis, tumour promotion and invasiveness, and UV-induced damage; (ii) protection of cardiac tissue against post-ischemia reperfusion damage; (iii) as an antiinflamatory agent; (iv) to reduce the cytotoxic and cardiotoxic effects of anticancer drugs; (v) endothelial disorders; (vi) degenerative diseases; (vii) coagulation disorders, and; (viii) to improve the longevity of living cells. Indeed, dysfunction of SOD boosts prematurely the ageing of cells and their apoptosis. Moreover, currently bovine Cu/ZnSOD is being utilised for the treatment of inflamed tendons in horses and for treating osteoarthritis in man. It has been shown that the mitochondrial antioxidant enzyme manganese-containing superoxide dismutase (MnSOD) functions as a tumor suppressor gene and that reconstitution of MnSOD expression in several human cancer cell lines leads to reversion of malignancy.

Size, antigenicity and cost, however, mitigate against their widespread usage. Since the enzyme must be isolated from biological sources or be prepared by genetic engineering, it is in limited supply, very expensive, and/or plagued by problems caused by contaminants. SODs currently proposed for therapy suffer the severe disadvantage of being highly immunogenic and consequently, as a result of the antibody response produced on administration, have proved to be of low clinical utility. Further available SODs, particularly those from mammalian sources, are difficult to obtain in large amounts in view of their low concentration in mammalian cells and the tedious isolation procedures required to produce them in satisfactory levels of purity.

It has long been apparent that mimics of SOD, capable of acting intracellularly, would be useful. Manganese (II), per se, will scavenger $O_2^-$ and, in suitable buffers, will do so catalytically. However, Mn(II) binds avidly to a number

of proteins and in so doing loses its activity. Moreover, no clinical data have shown so far that organic or mineral salt of manganese per se presents any beneficial, biological or therapeutic, effect, such as an antioxidative effect, on animals or humans at non-lethal doses. Cu(II) is itself a very effective catalyst of the dismutation Of $O_2^-$. Since the first SOD to be discovered was a copper protein, copper-complexes have been examined for SOD activity. The problems with free Cu(II) are that it readily forms a hydroxide and that it binds strongly to many macromolecules. For these reasons Cu(II) per se is most active in acid solutions and in the absence of strongly binding ligands. Among the complexes of Cu(II), the SOD-like activity which have been reported, are: Cu(lys)2 and Cu(gly-his)2, Cu(diisopropylsalicylate)2, Cu(penicillamine), Cu(histidine), Cu(dipeptides) and Cu(gly-his-1ys). There are serious problems with all of these copper complexes. Many are merely acting as metal buffers, serving to solubilize the Cu(II) and are of insufficient stability to retain activity in the presence of serum albumin. Investigations of Cu(II) complexes have thus far not resulted in the discovery of any biologically useful mimics of SOD.

## SUMMARY OF THE INVENTION

It is an object of the invention to provide an inexpensive anti-oxidative agent.

It is another object of the invention to provide an inexpensive and synthetic mimic of SOD.

It is a further object of the invention to provide a scavenger of superoxide radicals that is not inactivated by proteins.

It is another object of the invention to provide a method of using a mimic of SOD to reduce or prevent the toxicity of superoxide radical-induced toxicity.

It is a further object of the invention to provide a pharmaceutical composition containing, as an active ingredient, a stable mimic of superoxide dismutase.

Further objects and advantages of the present invention will be apparent from the following detailed description thereof.

The present invention relates mores specifically to organometallic complexes based on manganese, sterols and diglycerides as novel industrial products and uses thereof as a therapeutic or dietetic product, in particular as anti-oxidative agent.

The invention relates to pharmaceutical compositions and dietary products containing these complexes.

US 6,129,924 describes organometallic complexes which are obtainable by reaction:

- of a cation of a metal (M) in an oxydation state at least equal to 2 useful as a biocatalyst in living metabolism,
- of sitosterol, of sitostanol, or of a mixture of sitosterol and a sitostanol, or of a plant extract containing the same,
- of the diglyceride of formula :

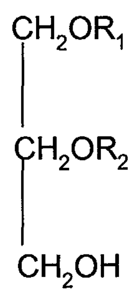

in which:

- $R_1$ is an acyl residue of oleic acid ($C_{18}$:1)
- $R_2$ is an acyl residue of a linear or branched, saturated or unsaturated fatty acid having between 2 and 18 carbon atoms.

The complexes described in this reference are more particularly the complexes in which the metal cation is vanadium, which have been proved to be particularly interesting for the treatment and/or prevention of insulin dependent or non-insulin dependent diabetes, of the cardiovascular complications thereof and/or of insulin resistance and of the cardiovascular complications thereof, and the treatment or the prevention of hypercholesterolaemia and/or hypertriglyceridaemia.

## DETAILLED DESCRIPTION OF THE INVENTION

In a first aspect, the invention, according to one of its essential characteristics, relates to the use of organometallic complexes obtainable by reaction of :

- a mineral or organic salt of manganese (including more specifically a cation of a manganese, in particular in an oxidation state of 2, 3, 4, 6 or 7),
- sitosterol, or a mixture of sitosterol and sitostanol, or a plant extract containing the same,
- the diglyceride of formula (I) :

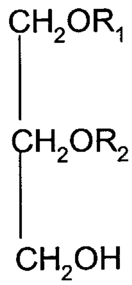

in which:

- $R_1$ is an acyl residue of oleic acid ($C_{18}$:1),
- $R_2$ is an acyl residue of a linear or branched, saturated or unsaturated fatty acid having between 2 and 18 carbon atoms,

for the preparation of a pharmaceutical composition for the treatment of a disease involving an oxidative stress.

In a particular aspect of the invention, the pharmaceutical composition is used to prevent or treat pathological conditions resulting from the presence of superoxide radicals.

The present invention also includes methods of treating diseases involving an oxidative stress or resulting from the presence of superoxide radicals, comprising the administration to a subject (animal or human) in need thereof of an effective amount of a complex as defined above.

In a preferred embodiment, the invention deals with complexes in which $R_2$ is an oleoyl or acetyl group.

The present invention deals therefore with organometallic manganese based complexes presenting a biological activity analogous to SOD. The complexes according to the invention have the advantage to be effective even at a low dose of manganese. These complexes have, when administered to animals, an anti-oxidative specific activity analogous to SOD on the same model.

This property is all the more important because in general, the person skilled in the art knows that the difficulty in the therapeutic use of metallic cations is linked to their toxicity at active doses. Moreover, it has never been reported that manganese per se could present clinically an anti-oxidative activity.

By way of examples, the following mineral or organic manganese salts or derivatives particularly useful according to the invention may be cited : manganese acetate, manganese carbonate, manganese oxyde, manganese sulfate, manganese ascorbate, manganese gluconate, manganese glycerophosphate, manganese oleate, manganese phosphate, or manganese citrate. More preferably, the manganese derivatives useful according to the invention are chosen in the group consisting of : manganese acetate, manganese carbonate, manganese oleate, manganese phosphate and manganese sulfate.

These derivatives are usually soluble in water or in organic solvents. They can be advantageously dissolved in water, or in alcoohols, preferably in ethanol.

The manganese derivatives to be used according to the invention can be extracted from foods or plants known to be rich in manganese as described above.

In a particular aspect, the invention relates to organometallic complexes obtainable by reaction of:

- at least one manganese derivative selected in the group consisting of manganese acetate, manganese carbonate, manganese oxyde, manganese sulfate, manganese ascorbate, manganese gluconate, manganese glycerophosphate, manganese oleate, manganese phosphate and manganese citrate,
- sitosterol, or a mixture of sitosterol and sitostanol, or a plant extract containing the same,

- the diglyceride of formula (I):

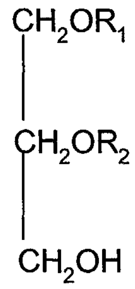

in which:

- $R_1$ is an acyl residue of oleic acid ($C_{18}$:1),
- $R_2$ is an acyl residue of a linear or branched, saturated or unsaturated fatty acid having between 2 and 18 carbon atoms.

More particularly, the complexes as described above are obtainable by reaction of diglyceride of formula (I) wherein R2 is oleoyl or acetyl group.
Sitosterol will preferably be used pure, however commercial products are a mixture with 85% of sitosterol and 15% of sitostanol, and sometimes with campesterol. These commercial products are generally extracted from soja or tall-oil.
The activity seemed to be linked exclusively to the presence of sitosterol, since there existed a dose-response relationship between the activity and the amount of sitosterol. Moreover, pure campesterol or sitostanol showed no activity.
Sitosterol and thus sitostanol can also be prepared by extraction from plants according to the techniques in the literature, for example p. 95 of the thesis presented at Montpellier by Claude Cerdon entitled «Modulation de la production de sapogenines steroidiques en reponse a l'inhibition de la synthese de sterols».
This extraction is carried out advantageously by complexation with metals, according to the method described in particular in French patent FR 2 316 247 in which is described a method for isolating 3-hydroxy-steroids and 3-oxo-steroids from a mixture containing these compounds.
To effect this extraction any plant or product of plant origin known for its relatively high content of sitosterol can be used.
By way of examples of plants or products of plant origin with a relatively high free sitosterol content may be mentioned in particular olive oil, soya bean oil, cotton leaves, coffee leaves, wheatgerm.
It must also be pointed out that the free sterol fraction contains a proportion of the 24 R and 24 S isomers, which are variable according to the plant. This proportion is not well known, since it has been little studied, if at all.
This proportion as well as the relative quantity of sitostanol, which cannot be separated from the sitosterol during purification, could explain the better relative activity of the sterol fraction of some plants, and especially the excess of sitosterol necessary for the preparation of the novel products described in the invention.
The diglyceride of formule (I), and more particularly those which are found to be the most active, exist commercially. This is the case particularly for 1-oleoyl-2-acetylglycerol and for 1,2-dioleoylglycerol, which exist as commercial products with a purity of about 98%. 1-Oleoyl-2-acetylglycerol is, in particular, obtained by chemical or biochemical synthesis, in particular by acetylation in the 2- position by acetyl CoA of 1,2-dioleoylglycerol, or even from a dioleoylphospholipid, in particular phosphatidylcholine which is present in olive oil and is broken down by phospholipase C into a diolein and a phosphorylcholine.
Furthermore, acylglycerols useful for the preparation of the complexes according to the invention can be isolated from some vegetable oils.
As a general rule, an oil containing a high concentration of oleic acid will be chosen as a source of acylglycerols according to the invention.
As examples of such oils may be quoted:

- Olive oil, where the content of oleic acid ($C_{18:1}$) is between 60 and 80%; European oils (Spain, Italy, South of France) being richer in $C_{18:1}$ than oils from North Africa.

- Sunflower oil, of the variety known as oleic sunflower hybrid, which contains 80% of $C_{18:1}$ instead of 16% in normal sunflower oil.
- Almond oil containing 64 to 82% of $C_{18:1}$.
- Hazel nut oil containing 66 to 83% of $C_{18:1}$.
- Avocado oil containing 36 to 80% of $C_{18:1}$.

The complexes according to the invention are easily prepared by simply mixing together the three types of compounds described above. The mixture is advantageously effected in an organic solvent, preferably ethanol. The mixture is more specifically stirred and kept several minutes (30 to 60 minutes) at room temperature or above (between 30 to 40 °C).

The complexes can also be prepared directly by using an oil as a solvent, which can subsequently be used as the injection solvent for the active product in the case of the injectable form, or as the excipient for the oral form.

As far as the proportions of the different constituents are concerned, they can vary over a wide range. Nevertheless by correlating the activity of the products obtained with the proportions of the three types of reagent used, it was possible to determine the following proportions, data given with respect to one mole of manganese:

- Sitosterol is advantageously used in the proportion of 1000 to 10000 mole per mole;
- The diglyceride is advantageously used in the proportion of 10 to 100 mole per mole.

By way of example, for 50 mg of a mixture of sitosterol, and for an amount of manganese from 1 to 5 µg (expressed as the metal), 500 to 2500 µg of 1,2-dioleoylglycerol will preferably be used.

The different constituents can be identified by the appropriate analytical methods:

- Manganese by atomic absorption,
- Sitosterol by gas phase chromatography, HPLC or H NMR.
- Acylglycerol by HPLC, with a light diffusing detector, on a $C_{18}$ kromasil column, with an eluent composed of isocratic acetonitrile.

The mass peak of the complex is not detectable by the usual methods, such as chemical ionisation and electron impact, which may be explained by the fact that the complexes formed by these two constituents with the metal are generally unstable, like the majority of organometallic complexes having biological activity.

The organometallic complexes described in the present invention optimise the bioavailability of manganese, permitting its therapeutic use with low or no toxicity, which shows a considerable advantage with respect to the state of the art.

According to another of its aspects, the invention also relates to pharmaceutical compositions which contain at least one complex of manganese as defined previously, and a pharmaceutically acceptable vehicle, excipient or carrier.

As pharmaceutically acceptable excipient, vehicle or carrier, any excipient vehicle or carrier well-known to the person skilled in the art may be used. The complex may be formulated in various forms, including solid and liquid forms, such as tablets, gel, syrup, powder, aerosol, etc.

The compositions of this invention may contain physiologically acceptable diluents, fillers, lubricants, excipients, solvents, binders, stabilizers, and the like. The following can be cited as examples in a non limiting way: lactose, corn starch, glucose, sucrose, sweetening agents such as maltitol syrup, gum arabic, gelatine, carrhagenans, stearic acid, magnesium stearate, dextrin, maltodextrins, mannitol, talc, fats from natural origin, particularly oils of vegetable origin rich in unsaturated fatty acids and sterols. In particular, if eventually necessary, other additives well-known to the person skilled in the art such as stabilisers, drying agents, binders or buffers may be used.

The compositions of the invention can be administred according to various routes, via the oral route, with a buccal or enteric absorption, via the mucous membrane, subcutaneous, intramuscular route, or the transdermal route (as a patch or gel).

The dosages and dosage regimen in which the complexes are administered will vary according to the dosage form, mode of administration, the condition being treated and particulars of the patient being treated. Generally, the manganese can be administered to human or animal in an amount of about 1 to 200 micrograms per day. Accordingly, optimal therapeutic concentrations will be best determined at the time and place through routine experimentation.

In this regard, "treatment" or "treating" include both therapeutic and prophylactic treatments. Accordingly, the compounds may be used at very early stages of a disease, or before early onset, or after significant progression.

According to a specific embodiment, the invention relates to the use of complexes as defined above for the preparation of a pharmaceutical composition for the prevention or the treatment of a disease involving an oxidative stress.

A further object of the invention is based on the use of complexes as defined above for the preparation of a pharmaceutical composition for the prevention or the treatment of a disease resulting from the presence of superoxide radicals.

Another aspect of the invention is the use of the complexes as a SOD like.

In this respect, they can be used to obtain the following therapeutic effects : (i) prevention of oncogenesis, tumour promotion and invasiveness, and UV-induced damage; (ii) protection of cardiac tissue against post-ischemia reperfusion damage; (iii) as an anti-inflammatory agent; (iv) to reduce the cytotoxic and cardiotoxic effects of anticancer drugs; (v) endothelial disorders; (vi) degenerative diseases; (vii) coagulation disorders, and; (viii) to improve the longevity of living cells.

In a particular embodiment, the diseases to be treated according to the invention can be selected in the group consisting of cancers, neurodegenerative diseases, retinal degenerative diseases, Age-Related Macular Degeneration (ARMD), ischemic brain injury, endothelial dysfunctions or disorders, diseases or disorders presenting an inflammatory condition, frostbite, myocardial infarction, rheumatoid arthritis, osteoarthritis, alcohol-induced liver damage, and coagulation disorders. The complexes can also be used to improve the longevity of living cells.

In particular, the complexes of the invention can be used to treat cancers, including colon or colo-rectal cancer, breast cancer, pleural mesothelioma, leukemias, glyoblastom, etc.

In another aspect, the complexes of the invention can be used to treat neuro-degenerative diseases, such as Parkinson disease, Huntington Chorea, and amyotrophic lateral sclerosis (ALS), etc.

According to another embodiment, the invention relates to dietetic products comprising the complex as defined above. This dietetic product comprises preferably the complex as defined above, wherein the diglyceride is comprised within a vegetable oil, in particular an oil containing a high content of oleic acid.

The dietetic products as defined above is more specifically useful to ensure maintenance of good health, in particular to improve the longevity of living cells and/or to prevent early apoptosis of cells.

The following examples are given solely as illustration of the invention.

## LEGEND TO THE FIGURES

Figure 1 : Paw volume values for each group treated with a complex according to the invention or Diosmine (see Tables 1, 2, 3).

Figure 2 Percentage of paw oedema, for each group treated with a complex according to the invention and Diosmine, together with standard deviation (see Tables 4, 5, 6).

Figure 3 Percentage of oedema inhibition (protection conferred by a complex according to the invention compared to protection conferred by Diosmine); see Table 7.

## EXAMPLES

Example 1 : Preparation of a complex according to the invention

1 g of commercial sitosterol dissolved in 10 ml of ethanol.
100 mg of a commercial mixture of 1,2-diolein and 1,3 diolein.
313 µg of manganese sulfate monohydrate.
20 ml of soja oil.

The mixture is stirred and heated 30 minutes. Ethanol is evaporated.

Example 2: Preparation of a complex according to the invention

1 g of commercial sitosterol dissolved in 10 ml of ethanol.
50 mg of 1,2-diolein.
626 µg of manganese sulfate monohydrate.
5 ml of olive oil.

The mixture is stirred and heated 30 minutes at 35 °C. Ethanol is evaporated.

Example 3: Preparation of a pharmaceutical composition according to the invention

A pharmaceutical composition is prepared from the complex formed according to example 2. The product is introduced into gastro-resistant capsules. The product may then be administered.

Example 4: Preparation of a pharmaceutical composition according to the invention

A pharmaceutical composition is prepared from the complex formed according to example 1. The product is liquid and is ready to be absorbed per os, or via the IP route in animals, in particular humans.

Example 5: Preparation of a dietary product for medical use according to the invention

20 g of commercial sitosterol dissolved in 50 ml of ethanol.
6,26 mg of manganese sulfate monohydrate.
120 ml of olive oil (extra virgin).
The mixture is stirred and heated 30 minutes at 35 °C.
The complex according to the invention is formed, and the product is ready to be absorbed per os.

Example 6: Pharmacological tests

The complex according to example 4 is evaluated according to the following procedure:

Pharmacological model

The adriamycin test is a specific test used to show evidence of the anti-free radical activity of a test substance. This test is described in G.Jadot, OEdema from adriamycin in the Wistar rat. SuperOxide Dismutases, p 73-77, Masson Ed., Paris, 1988.

1- Principle

One hour following injection of Adriamycin, a temporary inflammatory response lasting 12 hours can be observed. This first phase corresponds to the release of histamine, serotonine and the accumulation of polynuclear neutrophiles. A second inflammatory phase commences on the third day following administration and continues for 5 days. During this inflammatory phase, vascular hyperpermeability results in endothelial cell membrane alteration produced by free radical oxidative damage. Steroidal and non-steroidal anti-inflammatory drugs are not effective during this second inflammatory phase.
Research into an anti-free radical activity in the rat by intra-peritoneal administration consists of measuring the reduction in oedema produced by injection of adriamycin under the arch of the hind paw of the rat.

2- Animals

Male Wistar rats were used for these tests, after a quarantine period of 15 days.

3- Experimental protocol

OEdema was induced by subcutaneous injection of a 0.2ml solution of adriamycin (2,5 mg/ml) in the arch of the right hind paw of the rat (Siegel's method modified by Jadot (1988)).
The compounds tested were administered 2 hours before the first measurements on the first day, and 1 hour before the measurements on the following days, until the 8th day.
The measurement of oedema volume was determined by plethysmography and was performed immediately before injection of adriamycin on the first day (base measurement), and then every day at the same time.
The treatment was maintained for the entire duration of the study. The control group received the dosing vehicle only.

4- Treatment

Following the establishment of groups of 10 animals, daily intra-peritoneal administration was carried out according to the following scheme:

**Negative control:** animals administered olive oil (1 ml/kg).
**Positive control:** animals administered 100mg/kg de diosmine (1 ml/kg).

**Example 4:** animals administered example 4 (1 ml/kg)

(Diosmine is a plant flavenoid which possesses a vascular and perivascular anti-free radical activity).

5-Expression of results

Paw oedema, expressed as a percentage, is calculated for each animal at each time point :

$$\% \text{ oedema} = \frac{\text{Paw volume - paw volume (pre-treatment)}}{\text{paw volume (pre-treatment)}} \times 100$$

The percentage reduction in paw oedema at each time point and for each group of animals are calculated as follows :

$$\% = \frac{\text{OEdema volume (control) - OEdema volume (treated)}}{\text{OEdema volume (control)}} \times 100$$

Anti-free radical activity is shown by a significant reduction in paw volume in the treated group compared to the control group.

6- Results

6-1. Paw volume (Figure 1)

Individual and mean paw volume values for each group and time period are shown in the tables below.

TABLE 1:

| CONTROL : Olive oil (1 ml/kg IP) | | | | | | | |
|---|---|---|---|---|---|---|---|
| **D1 (base** | **D2** | **D3** | **D4** | **D5** | **D6** | **D7** | **D8** |
| 110 | 132 | 195 | 208 | 208 | 198 | 188 | 171 |
| 113 | 127 | 191 | 195 | 193 | 174 | 167 | 159 |
| 107 | 153 | 223 | 217 | 205 | 206 | 200 | 185 |
| 121 | 137 | 201 | 218 | 197 | 208 | 195 | 182 |
| 111 | 127 | 168 | 175 | 184 | 181 | 174 | 162 |
| 94 | 122 | 181 | 172 | 164 | 158 | 162 | 146 |
| 121 | 140 | 205 | 215 | 208 | 204 | 188 | 164 |
| 102 | 123 | 170 | 203 | 194 | 188 | 180 | 155 |
| 96 | 122 | 196 | 200 | 210 | 195 | 176 | 162 |
| 118 | 140 | 198 | 218 | 258 | 204 | 193 | 176 |
| **109.30** | **132.30** | **192.8** | **202.10** | **202.10** | **191.60** | **182.30** | **166.20** |
| **9.64** | **10.13** | **16.49** | **17.08** | **24.08** | **16.33** | **12.53** | **12.24** |

TABLE 2:

| POSITIVE CONTROL : DIOSMINE (100 mg/kg IP) | | | | | | | |
|---|---|---|---|---|---|---|---|
| **D1 (base)** | **D2** | **D3** | **D4** | **D5** | **D6** | **D7** | **D8** |
| 113 | 135 | 160 | 158 | 175 | 172 | 166 | 164 |

TABLE 2: (continued)

| POSITIVE CONTROL : DIOSMINE (100 mg/kg IP) | | | | | | | |
|---|---|---|---|---|---|---|---|
| **D1 (base)** | **D2** | **D3** | **D4** | **D5** | **D6** | **D7** | **D8** |
| 107 | 118 | 160 | 142 | 186 | 199 | 172 | 175 |
| 102 | 112 | 138 | 223 | 165 | 167 | 164 | 164 |
| 103 | 110 | 134 | 187 | 173 | 183 | 173 | 159 |
| 91 | 105 | 161 | 152 | 170 | 158 | 150 | 154 |
| 116 | 129 | 162 | 152 | 195 | 193 | 195 | 180 |
| 108 | 152 | 181 | 121 | 200 | 194 | 176 | 173 |
| 102 | 112 | 142 | 180 | 152 | 153 | 148 | 145 |
| 105 | 107 | 126 | 143 | 172 | 179 | 174 | 156 |
| 120 | 128 | 158 | 172 | 188 | 177 | 164 | 165 |
| **106.70** | **120.80** | **152.20** | **163.00** | **177.6** | **177.50** | **168.20** | **163.50** |
| **8.25** | **14.96** | **16.61** | **28.71** | **14.58** | **15.38** | **13.46** | **10.58** |

TABLE 3:

| Example 4 (1 ml/kg IP) | | | | | | | |
|---|---|---|---|---|---|---|---|
| **D1 (base)** | **D2** | **D3** | **D4** | **D5** | **D6** | **D7** | **D8** |
| 106 | 124 | 176 | 195 | 162 | 158 | 158 | 152 |
| 104 | 105 | 160 | 184 | 153 | 173 | 158 | 155 |
| 114 | 148 | 183 | 157 | 234 | 222 | 212 | 190 |
| 108 | 115 | 154 | 163 | 164 | 170 | 154 | 144 |
| 87 | 98 | 124 | 160 | 146 | 165 | 156 | 150 |
| 94 | 102 | 140 | 178 | 160 | 167 | 162 | 148 |
| 108 | 116 | 140 | 166 | 118 | 139 | 130 | 138 |
| 96 | 108 | 163 | ND* | 176 | 168 | 164 | 156 |
| 100 | 118 | 135 | 153 | 148 | 168 | 165 | 165 |
| 106 | 121 | 165 | 185 | 175 | 173 | 156 | 157 |
| **102.30** | **115.20** | **154.00** | **171.20** | **163.60** | **170.30** | **161.50** | **155.50** |
| **8.03** | **14.22** | **13.15** | **17.47** | **29.81** | **20.73** | **20.28** | **14.22** |

ND* = not determined

No statistical difference was observed between the base values (pre-treatment). The groups of animals are therefore identical.

The groups treated with Example 4 and Diosmine present no statistically significant difference in paw volume during the entire duration of the experiment. However, in comparison to the negative control, the groups treated with Example 4 and Diosmine show a statistically significant difference from D2-D7, with the highest significance for Example 4 at D3 and D4 ($p<0.001$).

The difference between the control and treated groups disappears at day 8 (D8).

6-2. Percentage oedema (Figure 2)

The individual and mean values expressed as a %, for each group, together with standard deviations, are shown in the table below.

TABLE 4:

| CONTROL : olive oil (1 ml/kg IP) | | | | | | |
|---|---|---|---|---|---|---|
| **D2** | **D3** | **D4** | **D5** | **D6** | **D7** | **D8** |
| 20.00 | 77.28 | 89.09 | 89.09 | 80.00 | 70.91 | 55.45 |
| 12.39 | 69.03 | 72.57 | 70.80 | 53.98 | 47.79 | 40.71 |
| 42.99 | 108.41 | 102.80 | 91.59 | 92.52 | 86.92 | 72.90 |
| 13.22 | 66.12 | 80.17 | 62.81 | 71.90 | 61.16 | 50.41 |
| 14.41 | 51.35 | 57.66 | 65.77 | 63.06 | 56.76 | 45.95 |
| 29.79 | 92.55 | 82.98 | 74.47 | 68.09 | 72.34 | 55.32 |
| 15.70 | 69.42 | 77.69 | 71.90 | 68.60 | 55.37 | 35.54 |
| 20.59 | 66.67 | 99.02 | 90.20 | 84.31 | 76.47 | 51.96 |
| 27.08 | 104.17 | 108.33 | 118.75 | 103.13 | 83.33 | 68.75 |
| 18.64 | 67.80 | 84.75 | 118.64 | 72.88 | 63.56 | 49.15 |
| **21.48** | **77.28** | **85.50** | **85.40** | **75.85** | **67.46** | **52.61** |
| **9.48** | **18.42** | **15.11** | **20.28** | **14.49** | **12.69** | **11.46** |

TABLE 5:

| POSITIVE CONTROL : DIOSMINE (100 mg/kg IP) | | | | | | |
|---|---|---|---|---|---|---|
| **D2** | **D3** | **D4** | **D5** | **D6** | **D7** | **D8** |
| 19.47 | 41.59 | 39.82 | 54.87 | 52.21 | 46.90 | 45.13 |
| 10.28 | 49.53 | 32.71 | 73.83 | 85.98 | 60.75 | 63.55 |
| 9.80 | 35.29 | 118.63 | 61.76 | 63.73 | 60.78 | 60.78 |
| 6.80 | 30.10 | 81.55 | 67.96 | 77.67 | 67.96 | 54.37 |
| 15.38 | 76.92 | 67.03 | 86.81 | 73.63 | 64.84 | 69.23 |
| 11.21 | 39.66 | 31.03 | 68.10 | 66.38 | 68.10 | 55.17 |
| 40.74 | 67.59 | 12.04 | 85.19 | 79.63 | 62.96 | 60.19 |
| 9.80 | 39.22 | 76.47 | 49.02 | 50.00 | 45.10 | 42.16 |
| 1.90 | 20.00 | 36.19 | 63.81 | 70.48 | 65.71 | 48.57 |
| 6.67 | 31.67 | 43.33 | 56.67 | 47.50 | 36.67 | 37.50 |
| **13.21** | **43.16** | **53.88** | **66.80** | **66.72** | **57.98** | **53.67** |
| **10.80** | **17.35** | **31.59** | **12.41** | **13.28** | **11.02** | **10.16** |

TABLE 6:

| Example 4 (1 ml/kg IP) | | | | | | |
|---|---|---|---|---|---|---|
| **D2** | **D3** | **D4** | **D5** | **D6** | **D7** | **D8** |
| 16.98 | 66.04 | 83.96 | 52.83 | 49.06 | 49.06 | 43.40 |
| 0.96 | 53.85 | 76.92 | 47.12 | 66.35 | 51.92 | 49.04 |
| 29.82 | 60.53 | 37.72 | 105.26 | 94.74 | 85.96 | 66.67 |
| 6.48 | 42.59 | 50.93 | 51.85 | 57.41 | 42.59 | 33.33 |

TABLE 6: (continued)

| Example 4 (1 ml/kg IP) | | | | | | |
|---|---|---|---|---|---|---|
| **D2** | **D3** | **D4** | **D5** | **D6** | **D7** | **D8** |
| 12.64 | 42.53 | 83.91 | 67.82 | 89.66 | 79.31 | 72.41 |
| 8.51 | 48.94 | 89.36 | 70.21 | 77.66 | 72.34 | 57.45 |
| 7.41 | 29.63 | 53.70 | 9.26 | 28.70 | 20.37 | 27.78 |
| 12.50 | 69.79 | ND* | 83.33 | 75.00 | 70.83 | 62.50 |
| 18.00 | 35.00 | 53.00 | 48.00 | 68.00 | 65.00 | 65.00 |
| 14.15 | 55.66 | 74.53 | 65.09 | 63.21 | 47.17 | 48.11 |
| **12.75** | **50.45** | **67.11** | **60.08** | **66.98** | **58.46** | **52.57** |
| **7.91** | **13.15** | **18.43** | **25.36** | **19.31** | **19.84** | **14.80** |

* ND not determined

The groups treated with Example 4 and Diosmine show no statistically significant difference in the percentage of paw oedema during the entire duration of the experiment.

However, the groups treated with Example 4 and Diosmine show a significant decrease in the percentage of paw oedema from D2 to D5 with the most statistical significant at D3 for

Example 4 ($p<0.001$) with regards to the maximum size of the oedema.

The difference between the percentage oedema between groups Example 4 and Diosmine disappeared on D6.

6-3. Percentage protection (Figure 3)

The percentages of oedema inhibition are shown in the following table and in the form of a graph.

TABLE 7:

| Treatment | **D3** | **D4** | **D5** | **D6** | **D7** | **D8** |
|---|---|---|---|---|---|---|
| **Diosmine** | 44.15 | 36.98 | 21.77 | 12.03 | 14.05 | 0 |
| **Example 4** | 37.71 | 21.50 | 29.64 | 11.69 | 13.34 | 0 |

Conclusion

Diosmine, the positive control, was used as a reference in order to show a protection effect regarding the development of oedema originating from free radical damage. This compound confirmed its activity with respect to oedema progression compared to the control group. Bovine superoxide dismutase possesses the same characteristics as Diosmine (cf. JL Jadot). The results obtained indicated that the product formulated according to example 4, possesses a free radical protection activity similar to compounds traditionally used as reference compounds, namely Diosmine and superoxide dismutase.

## Claims

**1.** Use of organometallic complexes obtainable by reaction of :

- a mineral or organic salt of manganese,
- sitosterol, or a mixture of sitosterol and sitostanol, or a plant extract containing the same,
- the diglyceride of formula (I) :

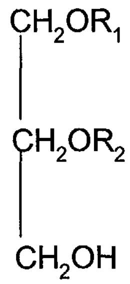

in which:

- $R_1$ is an acyl residue of oleic acid ($C_{18}$:1),
- $R_2$ is an acyl residue of a linear or branched, saturated or unsaturated fatty acid having between 2 and 18 carbon atoms,

for the preparation of a pharmaceutical composition for the treatment of a disease involving an oxidative stress.

**2.** Use according to claim 1, wherein the pharmaceutical composition is used to prevent or treat pathological conditions resulting from the presence of superoxide radicals.

**3.** Use according to claim 1 or 2, wherein $R_2$ is an oleic acid residue or acetyl residue.

**4.** Use according to claim 1, wherein the diglyceride is obtained by isolation from olive oil or an oil rich in oleic acid.

**5.** Use according to any one of the preceding claims, wherein the mineral or organic manganese salt is selected in the group consisting of : manganese acetate, manganese carbonate, manganese oxyde, manganese sulfate, manganese ascorbate, manganese gluconate, manganese glycerophosphate, manganese oleate, manganese phosphate, and manganese citrate.

**6.** Use according to any one of the preceding claims, wherein the pharmaceutical composition is used (i) for prevention of oncogenesis, tumour promotion and invasiveness, and UV-induced damage; (ii) for protection of cardiac tissue against post-ischemia reperfusion damage; (iii) as an anti-inflammatory agent; (iv) to reduce the cytotoxic and cardiotoxic effects of anticancer drugs; (v) to treat endothelial disorders; (vi) to treat degenerative diseases; (vii) to treat coagulation disorders, or ; (viii) to improve the longevity of living cells.

**7.** Use according to any one of the preceding claims, wherein the disease is selected in the group consisting of cancers, neurodegenerative diseases, retinal degenerative diseases, Age-Related Macular Degeneration (ARMD), ischemic brain injury, endothelial dysfunctions or disorders, diseases or disorders presenting an inflammatory condition, frostbite, myocardial infarction, rheumatoid arthritis, osteoarthritis, alcohol-induced liver damage, and coagulation disorders.

**8.** Use according to any one of the preceding claims, wherein the disease is selected in the group consisting of colon or colo-rectal cancer, breast cancer, pleural mesothelioma, leukemias, and glyoblastom.

**9.** Use according to any one of the preceding claims, wherein the disease is selected in the group consisting of Parkinson disease, Huntington Chorea, and amyotrophic lateral sclerosis.

**10.** Organometallic complexes obtainable by reaction of:

- at least one manganese derivative selected in the group consisting of manganese acetate, manganese carbonate, manganese oxyde, manganese sulfate, manganese ascorbate, manganese gluconate, manganese glycerophosphate, and manganese citrate,
- sitosterol, or a mixture of sitosterol and sitostanol, or a plant extract containing the same,
- the diglyceride of formula (I):

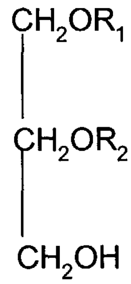

in which:

- $R_1$ is an acyl residue of oleic acid ($C_{18}$:1),
- $R_2$ is an acyl residue of a linear or branched, saturated or unsaturated fatty acid having between 2 and 18 carbon atoms.

**11.** Complexes according to the preceding claim, wherein R2 is oleoyl or acetyl group.

**12.** Complexes according to claim 10 or 11, wherein the diglyceride is obtained by isolation from olive oil or an oil rich in oleic acid.

**13.** Pharmaceutical composition comprising, in a pharmaceutically acceptable support, at least one complex as defined in any claim 10-12.

**14.** Pharmaceutical composition according to claim 13, for the treatment of a disease involving an oxidative stress.

**15.** Pharmaceutical composition according to claim 13, for preventing or treating pathological conditions resulting from the presence of superoxide radicals.

**16.** Pharmaceutical composition according to claim 13, (i) for prevention of oncogenesis, tumour promotion and invasiveness, and UV-induced damage; (ii) for protection of cardiac tissue against post-ischemia reperfusion damage; (iii) as an anti-inflammatory agent; (iv) to reduce the cytotoxic and cardiotoxic effects of anticancer drugs; (v) to treat endothelial disorders; (vi) to treat degenerative diseases; (vii) to treat coagulation disorders, or ; (viii) to improve the longevity of living cells.

**17.** Pharmaceutical composition according to claim 13, for treating cancers, neurodegenerative diseases, retinal degenerative diseases, Age-Related Macular Degeneration (ARMD), ischemic brain injury, endothelial dysfunctions or disorders, diseases or disorders presenting an inflammatory condition, frostbite, myocardial infarction, rheumatoid arthritis, osteoarthritis, alcohol-induced liver damage, or coagulation disorders.

**18.** Pharmaceutical composition according to claim 13, for treating colon or colo-rectal cancer, breast cancer, pleural mesothelioma, leukemias, or glyoblastom.

**19.** Pharmaceutical composition according to claim 13, for treating Parkinson disease, Huntington Chorea, or amyotrophic lateral sclerosis.

**20.** Dietary product containing at least one complex as defined in any claim 10-13.

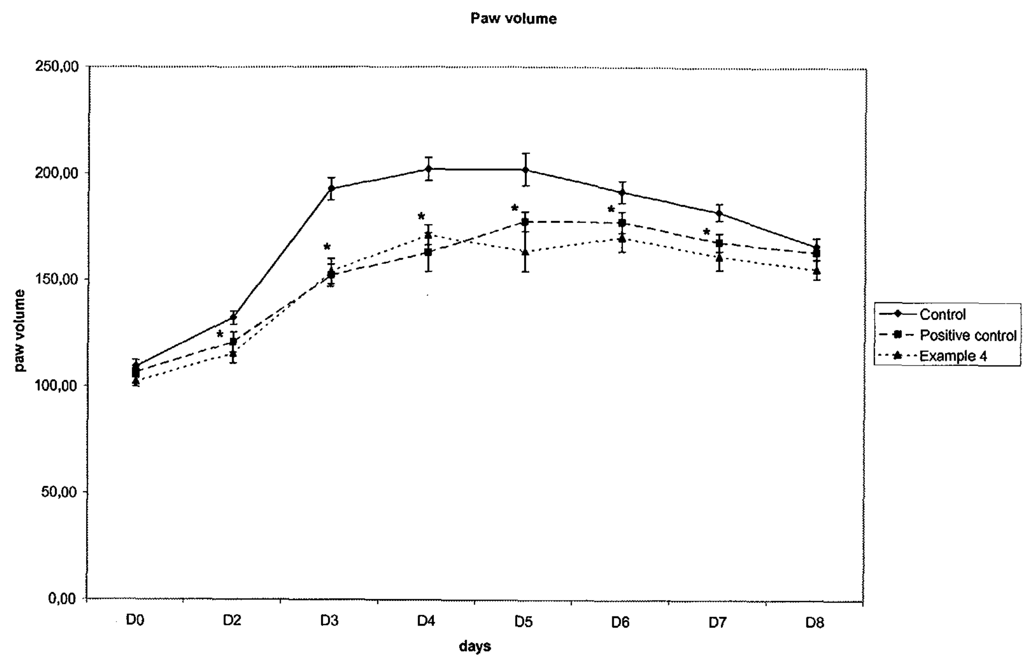
Paw volume
250,00
200,00
150,00
100,00
50,00
0,00
paw volume
D0
D2
D3
D4
D5
D6
D7
D8
days
Control
Positive control
Example 4
*

Figure 1

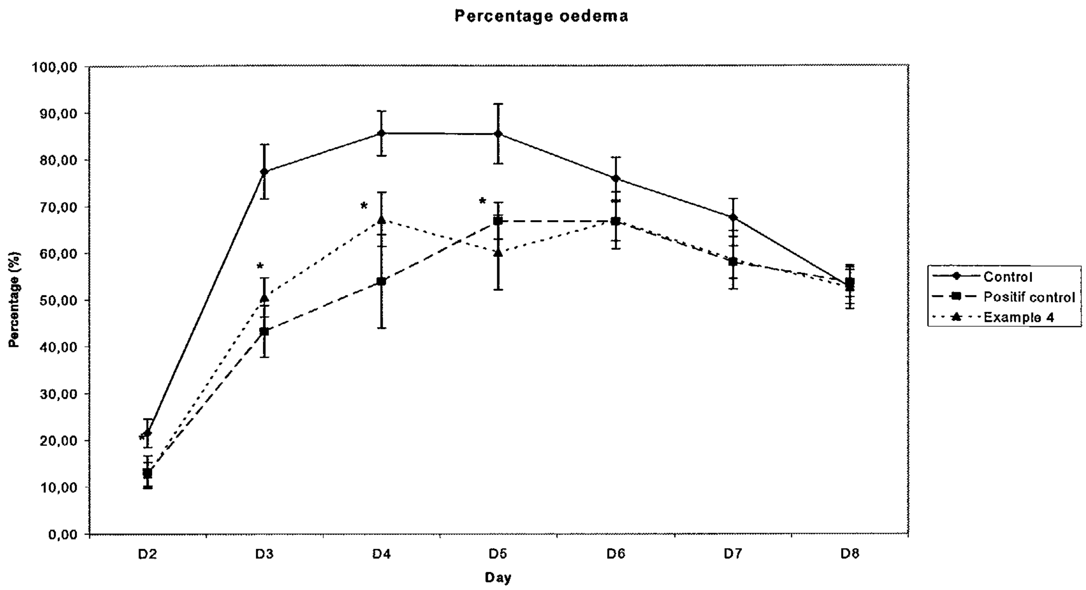

Percentage oedema
Percentage (%)
100,00
90,00
80,00
70,00
60,00
50,00
40,00
30,00
20,00
10,00
0,00
D2
D3
D4
D5
D6
D7
D8
Day
Control
Positif control
Example 4


Figure 2

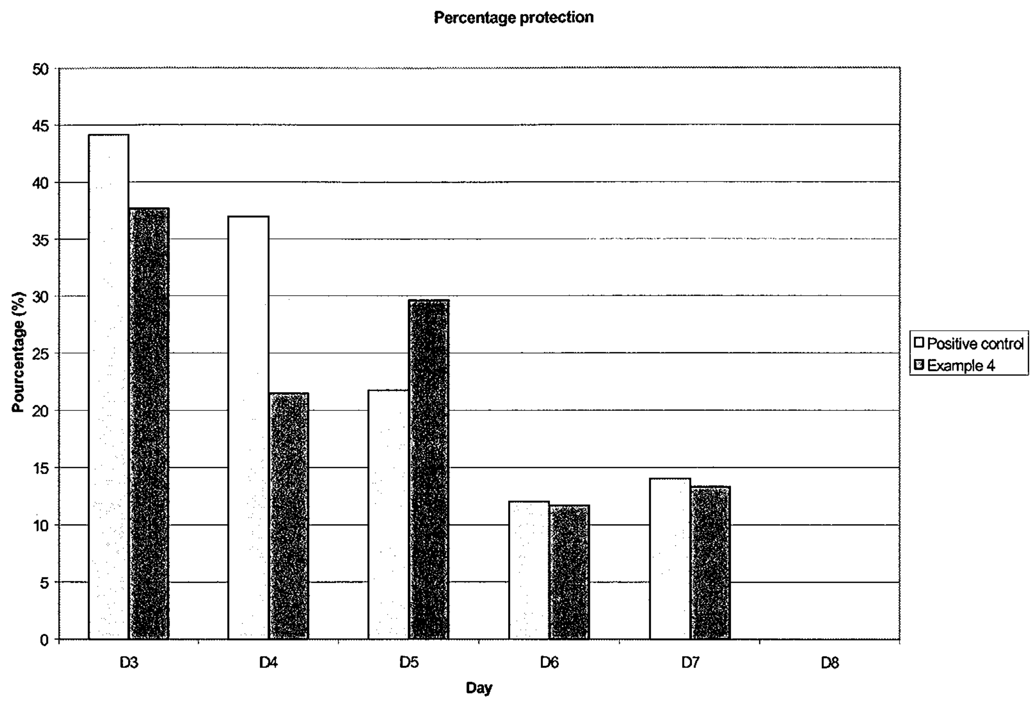
Percentage protection
50
45
40
35
30
25
20
15
10
5
0
Pourcentage (%)
D3
D4
D5
D6
D7
D8
Day
Positive control
Example 4

Figure 3

European Patent Office

# EUROPEAN SEARCH REPORT

Application Number
EP 03 29 0491

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | US 6 129 924 A (CHAPUIS JEAN-MARC ET AL) 10 October 2000 (2000-10-10)<br>* the whole document *<br>--- | 1-20 | A61K31/28<br>A61P39/06 |
| X | WO 02 36134 A (CROS GERARD ;RIBES GERARD (FR); MAUREL JEAN CLAUDE (FR); JASALI LT) 10 May 2002 (2002-05-10)<br>* the whole document *<br>--- | 1-20 | |
| X | US 5 714 143 A (ZOEPHEL ANDREAS ET AL) 3 February 1998 (1998-02-03)<br>* the whole document *<br>--- | 1-20 | |
| X | WO 96 23811 A (CHAPUIS JEAN MARC ;MAUREL SANTE (FR); SAENZ CHRISTIAN (FR); MAUREL) 8 August 1996 (1996-08-08)<br>* the whole document *<br>--- | 1-20 | |
| A | DUBICK M A ET AL: "SUPEROXIDE DISMUTASE ACTIVITY IN LUNG FROM COPPER AND MANGANESE-DEFICIENT MICE EXPOSED TO OZONE" TOXICOLOGY LETTERS (AMSTERDAM), vol. 42, no. 2, 1988, pages 149-157, XP009015233<br>ISSN: 0378-4274<br>* the whole document *<br>----- | 1-20 | TECHNICAL FIELDS SEARCHED (Int.Cl.7)<br>A61K<br>A61P |

The present search report has been drawn up for all claims

| Place of search | Date of completion of the search | Examiner |
|---|---|---|
| MUNICH | 19 August 2003 | Skjöldebrand, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 29 0491

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2003

| Patent document cited in search report | Publication date | Patent family member(s) | Publication date |
|---|---|---|---|
| US 6129924 A | 10-10-2000 | FR 2750606 A1 | 09-01-1998 |
| | | AT 209657 T | 15-12-2001 |
| | | AU 3449397 A | 02-02-1998 |
| | | DE 69708643 D1 | 10-01-2002 |
| | | DE 69708643 T2 | 01-08-2002 |
| | | EP 0912599 A1 | 06-05-1999 |
| | | ES 2170401 T3 | 01-08-2002 |
| | | WO 9801461 A1 | 15-01-1998 |
| WO 0236134 A | 10-05-2002 | GB 2368521 A | 08-05-2002 |
| | | AU 2068102 A | 15-05-2002 |
| | | WO 0236134 A1 | 10-05-2002 |
| US 5714143 A | 03-02-1998 | AT 209928 T | 15-12-2001 |
| | | AU 677215 B2 | 17-04-1997 |
| | | AU 4623793 A | 17-03-1994 |
| | | CA 2105612 A1 | 10-03-1994 |
| | | DE 59310242 D1 | 17-01-2002 |
| | | DK 587055 T3 | 02-04-2002 |
| | | EP 0587055 A1 | 16-03-1994 |
| | | ES 2169033 T3 | 01-07-2002 |
| | | JP 6192123 A | 12-07-1994 |
| | | PT 587055 T | 31-05-2002 |
| | | ZA 9306612 A | 01-03-1995 |
| WO 9623811 A | 08-08-1996 | FR 2729956 A1 | 02-08-1996 |
| | | FR 2729957 A1 | 02-08-1996 |
| | | AU 4667096 A | 21-08-1996 |
| | | WO 9623811 A1 | 08-08-1996 |

EPO FORM P0459

For more details about this annex : see Official Journal of the European Patent Office, No. 12/82